# EUROPEAN PATENT APPLICATION

(11) **EP 4 636 092 A1**
(43) Date of publication of application: **22.10.2025**
(21) Application number: 23903728.6
(22) Date of filing: 24.10.2023
(51) Int. Cl.: C12N 15/86, C12N 7/00

(54) **METHOD FOR PRODUCING HIGH YIELD AND HIGH QUALITY RECOMBINANT ADENO-ASSOCIATED VIRUS**

(30) Priority: 16.12.2022 KR 20220177009
(71) Applicant: Aavatar Therapeutics Co., Ltd., Hanam-si, Gyeonggi-do 12925 (KR)
(72) Inventor: CHO, Seunghee, Hanam-si, Gyeonggi-do 12929 (KR); HEO, Sunhak, Namyangju-si, Gyeonggi-do 12097 (KR); CHOI, Hyeonjae, Bucheon-si, Gyeonggi-do 14461 (KR)
(74) Representative: Dompatent Partnerschaft von Patent- und Rechtsanwälten mbB
(86) International application number: PCT/KR2023/016591
(87) International publication number: WO 2024/128524

(57) **Abstract**

The present specification provides a method for producing a recombinant adeno-associated virus (rAAV) of a desired composition in a high yield and a high quality. The method for producing a recombinant adeno-associated virus disclosed in the present application is characterized by comprising a pre-culture process and an osmotic shock process. The pre-culture process and the osmotic shock process synergize with each other within the production method to produce recombinant adeno-associated virus in high yield and high quality.

## Description

### Technical Field

This specification discloses technical details on a method for preparing a recombinant adeno-associated virus.

### Background Art

The method of producing recombinant adeno-associated virus may be divided into the following processes: a) an upstream process of producing a recombinant adeno-associated virus through host cells; and b) a downstream process of harvesting, filtering, separating, and purifying the recombinant adeno-associated virus produced in the host cells. The upstream process is directly related to a process by which the host cells express each component of the recombinant adeno-associated virus. The downstream process is directly related to a method of separating and purifying only a target recombinant adeno-associated virus from a mixture of various biological materials such as proteins, nucleic acids, and lipids.

### Disclosure

### Technical Problem

The technical task of this specification is to provide a method for producing a recombinant adeno-associated virus (rAAV) of the desired composition in high yield and high quality.

### Technical Solution

To achieve the technical task, the method for producing a recombinant adeno-associated virus disclosed herein includes a pre-culture process and an osmotic shock process. The pre-culture process allows the host cells to produce a recombinant adeno-associated virus more efficiently and in greater quantities, thereby contributing to yield improvement. The osmotic shock process causes the host cells to release most of the produced recombinant adeno-associated virus into the media, allowing the adeno-associated virus to be recovered directly from the media without cell disruption. Due to the osmotic shock process, the recombinant adeno-associated virus can be recovered and purified without causing significant damage to cells. As a result, high-quality recombinant adeno-associated virus can be produced. Furthermore, due to the osmotic shock process, the recombinant adeno-associated virus production method can omit a process of cell disruption, thereby the adeno-associated virus purification and acquisition processes can be simplified. Accordingly, the production method of the present disclosure can help to achieve further improved yield and high quality by minimizing virus particles lost during the purification and acquisition.

The pre-culture process and the osmotic shock process create a synergy with each other within the production method to produce a recombinant adeno-associated virus in high yield and high quality.

### Advantageous Effects

By following the method of producing recombinant adeno-associated virus disclosed herein, recombinant adeno-associated virus can be produced in high yield and high quality. The method for producing a recombinant adeno-associated virus disclosed herein is a more excellent and economical method for producing a recombinant adeno-associated virus than the conventional production method.

### Description of Drawings

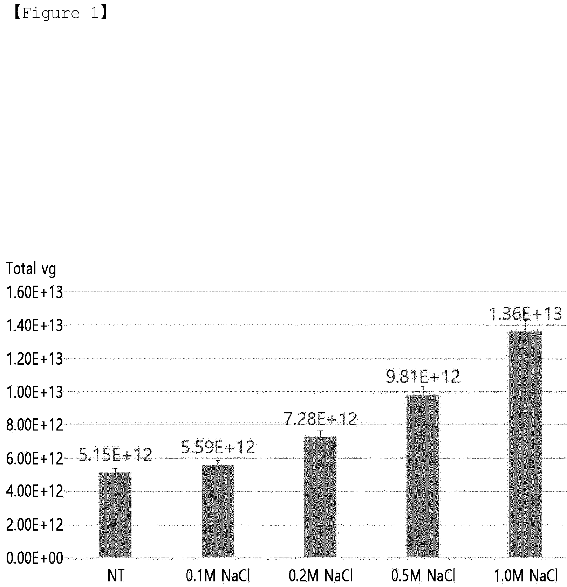

Figure 1 shows a graph showing a yield improvement effect of an osmotic shock process depending on salt concentration according to Experimental Example 2.2, after performing the osmotic shock process for each experimental group, a total number of viral genomes (vg) was measured and shown, herein, NT represents a negative control group in which the osmotic shock process was not performed, and NaCl was added to the remaining experimental groups at a corresponding concentration indicated on each label as an osmotic shock reagent;

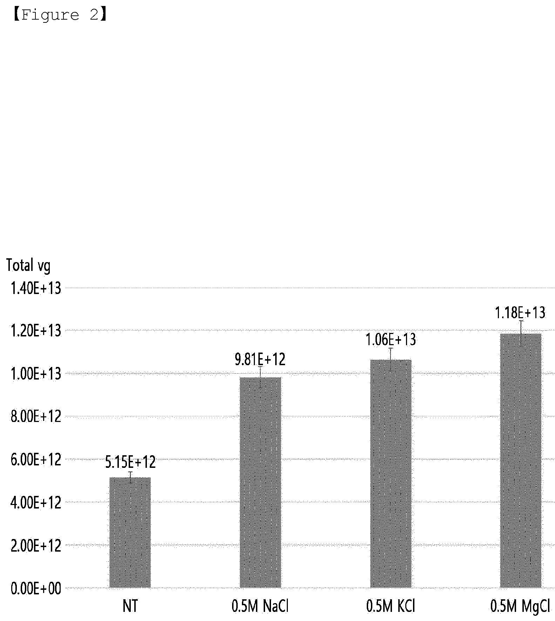

Figure 2 shows a graph showing the yield improvement effect of the osmotic shock process depending on the salt type according to Experimental Example 2.3, after performing the osmotic shock process for each experimental group, a total number of viral genomes (vg) was measured and shown, herein, NT represents a negative control group in which the osmotic shock process was not performed, and salts of the type and concentration indicated on each label were used for the remaining experimental groups as an osmotic shock reagent;

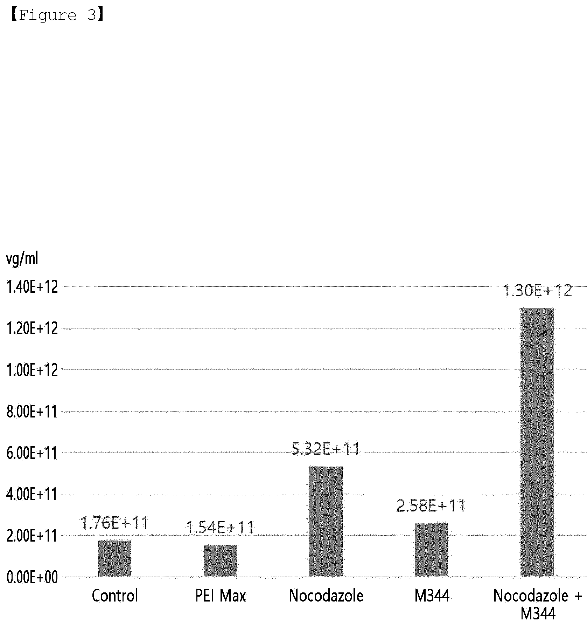

Figure 3 shows a graph showing the yield improvement effect of a pre-culture treatment process according to Experimental Example 3.2, after performing the pre-culture treatment process for each experimental group, a total number of viral genomes (vg) was measured and shown, herein, Control represents a negative control in which no pre-culture treatment process was performed, each type of material indicated on the label was used for the remaining experimental groups as a pre-culture treatment agent, and each detailed configuration is shown in Table 3 in a paragraph of (Experimental Example 3.2. Yield improvement Effect when Pre-culture Treatment Is Performed with Nocodazole and M344) ;

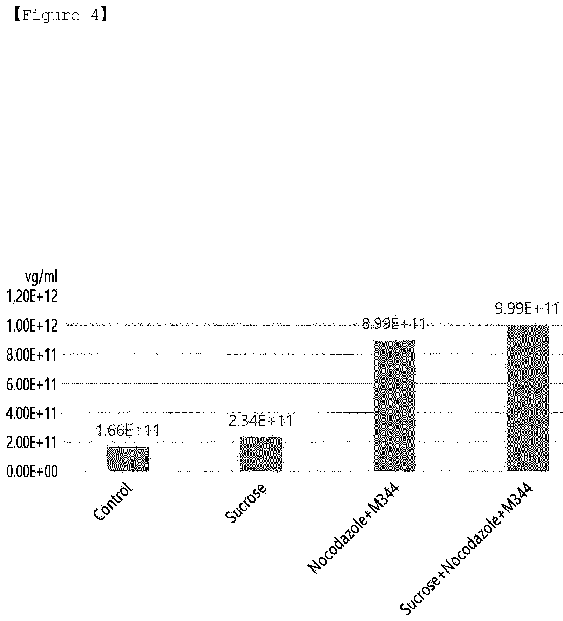

Figure 4 shows a graph showing the yield improvement effect of a pre-culture treatment process according to Experimental Example 3.3, after performing the pre-culture treatment process for each experimental group, a total number of viral genomes (vg) was measured and shown, herein, Control represents a negative control in which no pre-culture treatment process was performed, each type of material indicated on the label was used for the remaining experimental groups as a pre-culture treatment agent, and each detailed configuration is shown in Table 4 in a paragraph of «Experimental Example 3.3. Yield Improvement Effect when Pre-culture Treatment Is Performed with Nocodazole and M344» ;

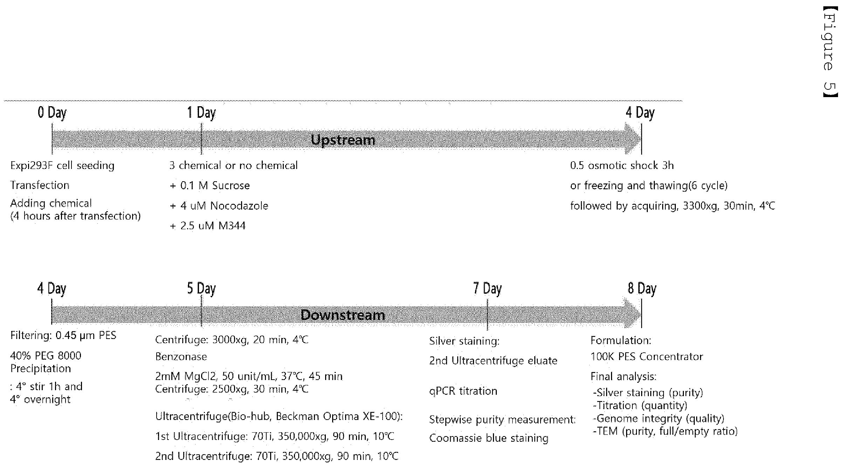

Figure 5 shows a schematic diagram schematically showing an entire production process disclosed herein;

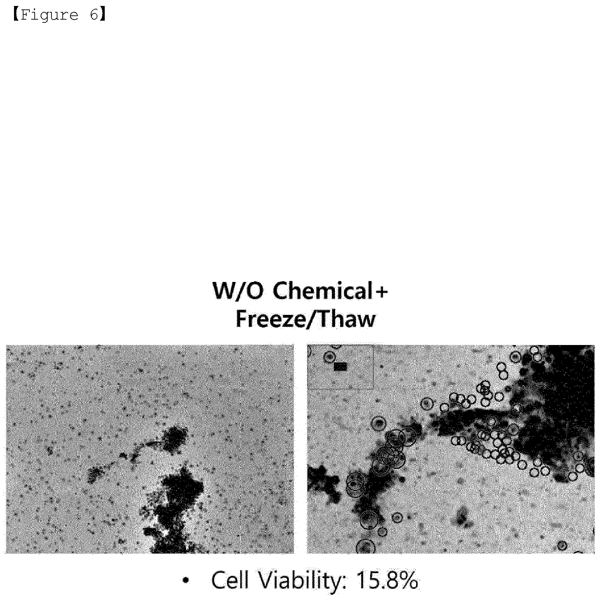

Figure 6 shows cell photos taken before performing a downstream process in Process 2 (not including a pre-culture treatment, including a freezing and thawing process) of Experimental Example 4, and as a result of measuring a cell survival rate of the process, it was measured to be 15.8%;

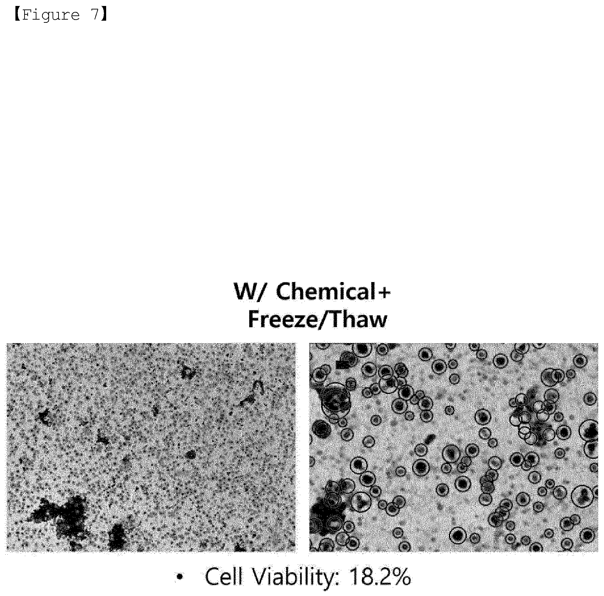

Figure 7 shows cell photos taken before performing a downstream process in Process 5 (including a pre-culture treatment, including a freezing and thawing process) of Experimental Example 4, and as a result of measuring a cell survival rate of the process, it was measured to be 18.2%;

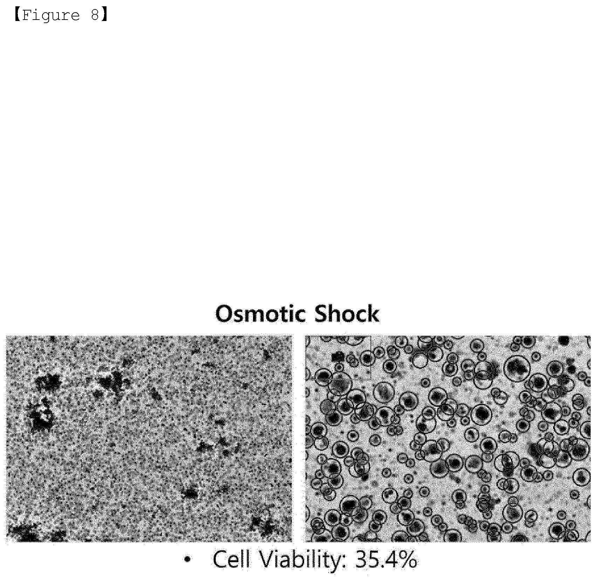

Figure 8 shows cell photos taken before performing a downstream process in Process 6 (including a pre-culture treatment, including an osmotic shock process) of Experimental Example 4, and as a result of measuring a cell survival rate of the process, it was measured to be 35.4%;

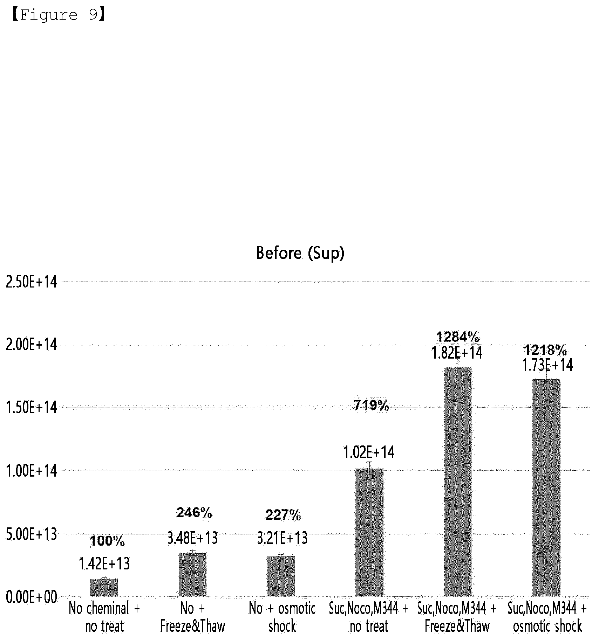

Figure 9 shows the number of viral genomes (vg) measured in media before performing the downstream process in each process of Experimental Example 4, herein, No chemical + no treat represents Process 1, No + Freeze&Thaw represents Process 2, No + osmotic shock represents Process 3, Suc,Noco,M344 + no treat represents Process 4, Suc,Noco,M344 + Freeze&Thaw represents Process 5, and Suc,Noco,M344 + osmotic shock represents Process 6, respectively;

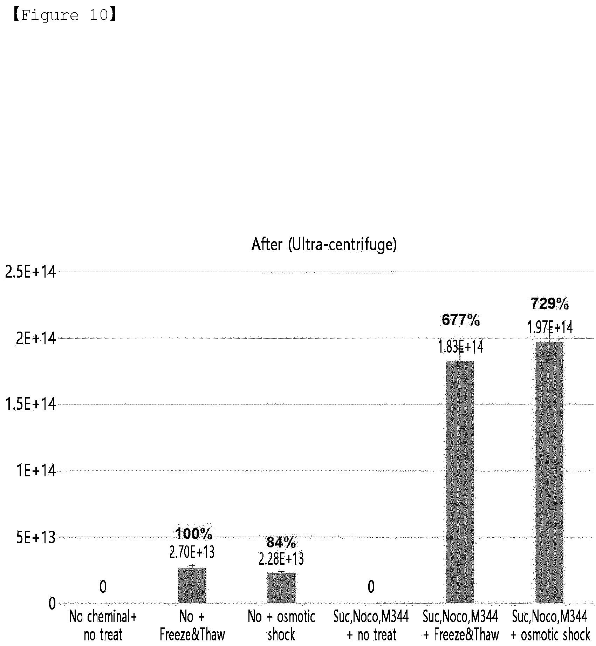

Figure 10 shows the final number of viral genomes (vg) acquired after ultra-centrifuge in each process of Experimental Example 4, herein, No chemical + no treat represents Process 1, No + Freeze&Thaw represents Process 2, No + osmotic shock represents Process 3, Suc,Noco,M344 + no treat represents Process 4, Suc,Noco,M344 + Freeze&Thaw represents Process 5, and Suc,Noco,M344 + osmotic shock represents Process 6, respectively;

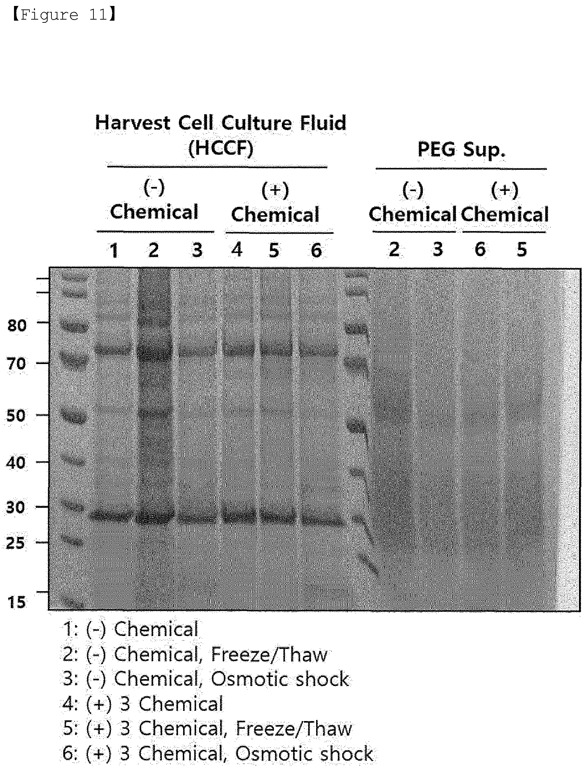

Figure 11 shows results of Coomassie brilliant blue staining after each process in each process of Experimental Example 4, herein, Harvest Cell Culture Fluid represents a sample at the time of harvesting the media before downstream processing, PEG Sup. represents a supernatant sample after filtration and PEG precipitation, in addition, 1: (-) chemical represents Process 1, 2: (-) Chemical and Freeze/Thaw represents Process 2, 3: (-) Chemical and Osmotic shock represents Process 3, 4: (+) 3 Chemical represents Process 4, 5: (+) 3 Chemical and Freeze/Thaw represents Process 5, and 6: (+) 3 Chemical and Osmotic shock represents Process 6, respectively;

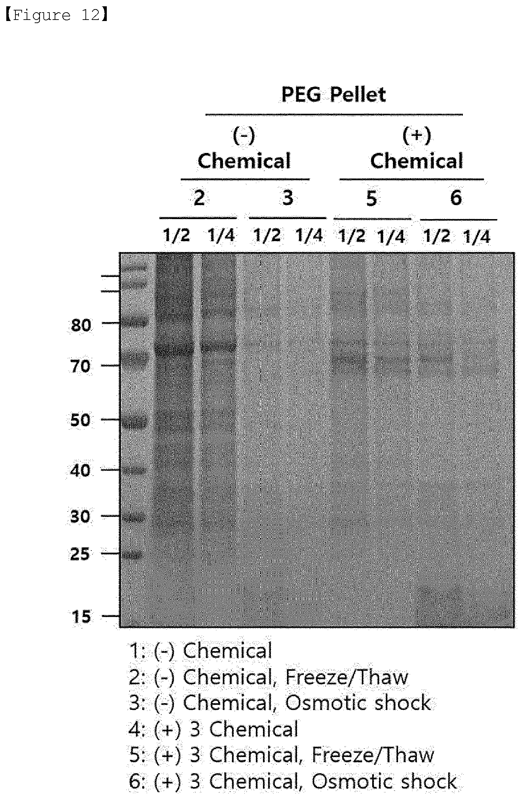

Figure 12 shows results of Coomassie brilliant blue staining of cell pellet samples after filtration and PEG precipitation in each process of Experimental Example 4, herein, 1: (-) Chemical represents Process 1, 2: (-) Chemical and Freeze/Thaw represents Process 2, 3: (-) Chemical and Osmotic shock represents Process 3, 4: (+) 3 Chemical represents Process 4, 5: (+) 3 Chemical and Freeze/Thaw represents Process 5, and 6: (+) 3 Chemical and Osmotic shock represents Process 6, respectively;

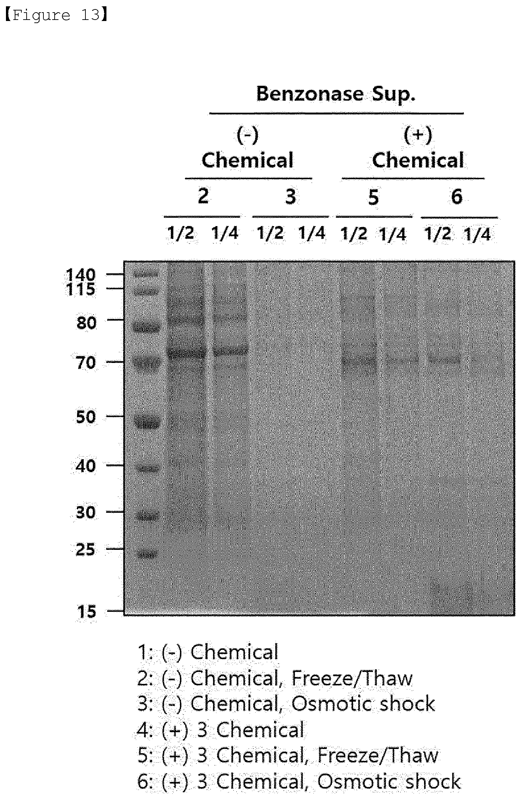

Figure 13 shows results of Coomassie brilliant blue staining of the supernatant samples after a filtration and benzonase process in each process of Experimental Example 4, herein, 1: (-) Chemical represents Process 1, 2: (-) Chemical and Freeze/Thaw represents Process 2, 3: (-) Chemical and Osmotic shock represents Process 3, 4: (+) 3 Chemical represents Process 4, 5: (+) 3 Chemical and Freeze/Thaw represents Process 5, and 6: (+) 3 Chemical and Osmotic shock represents Process 6, respectively;

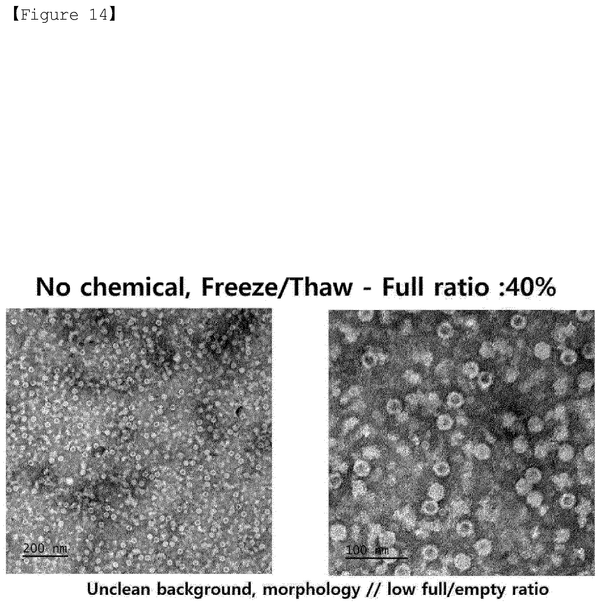

Figure 14 shows results of negative staining TEM photos taken according to Experimental Example 4.6 for Process 2 of Experimental Example 4, and as a result of photo-shooting, it was confirmed that a background was dirty, morphology of the virus particles was unclear, and a ratio of full capsid to empty capsid was 40%;

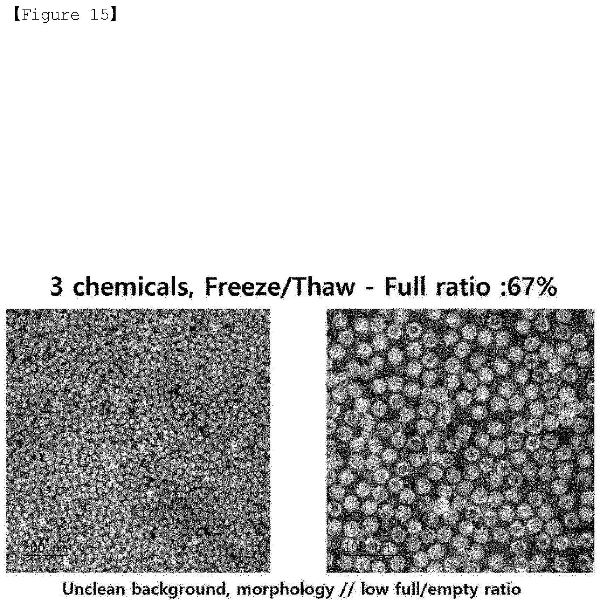

Figure 15 shows results of negative staining TEM photos taken according to Experimental Example 4.6 for Process 5 of Experimental Example 4, and as a result of photo-shooting, it was confirmed that a background was dirty, morphology of the virus particles was unclear, and a ratio of full capsid to empty capsid was 67%;

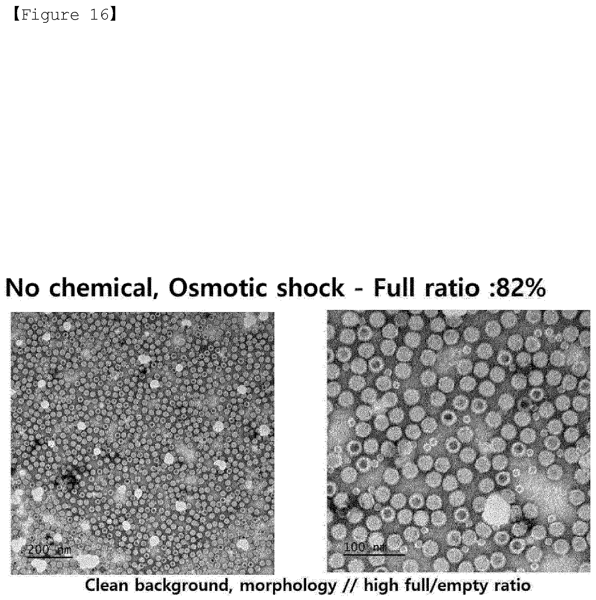

Figure 16 shows results of negative staining TEM photos taken according to Experimental Example 4.6 for Process 3 of Experimental Example 4, and as a result of photo-shooting, it was confirmed that a background was clear, morphology of the virus particles was clear, and a ratio of full capsid to empty capsid was 82%;

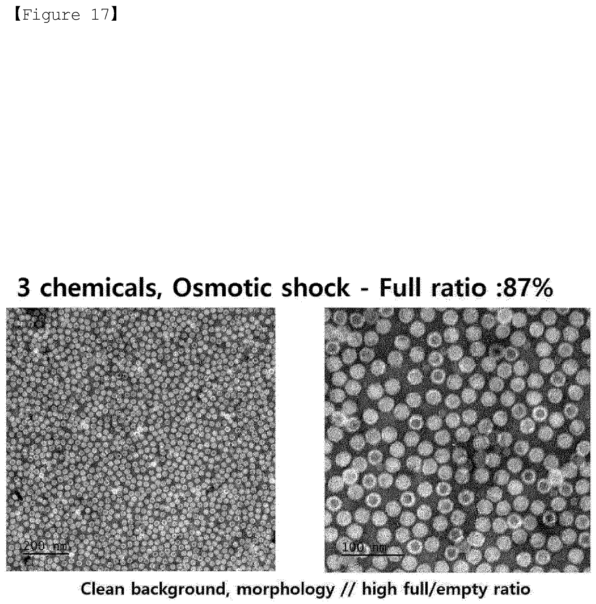

Figure 17 shows results of negative staining TEM photos taken according to Experimental Example 4.6 for Process 6 of Experimental Example 4, and as a result of photo-shooting, it was confirmed that a background was clear, morphology of the virus particles was clear, and a ratio of full capsid to empty capsid was 87%; and

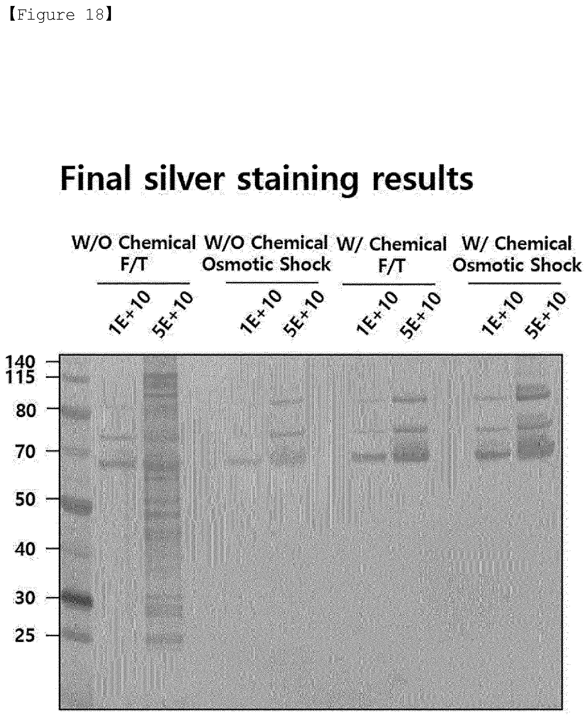

Figure 18 shows results of silver staining for a final sample acquired after performing all steps of each process in Experimental Example 4, herein, W/O Chemical F/T represents Process 2, W/O Chemical Osmotic Shock represents Process 3, W/ Chemical F/T represents Process 5, and W/ Chemical Osmotic Shock represents Process 6.

### Best Mode

Hereinafter, with reference to the attached drawings, the content of the present disclosure will be described in more detail through specific implementation embodiments and examples. It should be noted that the attached drawings include some, but not all, embodiments of the present disclosure. The subject matter of the present disclosure disclosed by this specification may be implemented in various ways and is not limited to the specific implementation examples described herein. These implementations should be viewed as being provided to satisfy the legal requirements applicable to this specification. Those skilled in the art will be able to think of many modifications and other implementations of the disclosure disclosed herein. Accordingly, it should be understood that the content of the present disclosure disclosed herein is not limited to the specific embodiments described herein, and that modifications and other embodiments thereof are also included within the scope of the claims.

This specification discloses a method for producing a recombinant Adeno Associated Virus (rAAV) comprising:
(a) preparing host cells,
   wherein the host cell comprises a vector encoding components of the rAAV,
   such that the host cells are capable of expressing the rAAV;
(b) adding sugar, Nocodazole and M344 to a medium comprising the host cells;
(c) culturing the host cells,
   wherein the culturing enables the expression of the rAAV within the host cells;
(d) applying an osmotic shock by adding salt to the media comprising the host cells,
   wherein the osmotic shock facilitates the release of the rAAV from the host cells into the medium; and
(e) obtaining the rAAV from the medium without lysing the host cells.

In one embodiment, a serotype of the rAAV is selected from one or more of the following:
wild-type serotypes including AAV1, AAV2, AAV3, AAV4, AAV5, AAV6, AAV7, AAV8, AAV9, and AAV10;
synthetic (variant) serotypes including AAV-DJ, AAV-DJ8, AAV-DJ9, and AAV6.2; 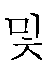
other AAV variant serotypes.

In one embodiment, the step (e) comprises:
(e-1) separating the medium from the host cells in the product of step (d);
(e-2) filtering the medium;
(e-3) performing a PET precipitation on the product of step (e-2);
(e-4) adding benzonase to the product of step (e-3) in the presence of a salt;
   (e-5) performing a 1st iodixanol gradient ultracentrifugation on the product of step (e-4); and
   (e-6) performing a 2nd iodixanol gradient ultracentrifugation on the product of step (e-5).

### Mode for Disclosure

### Term Definition

### About

As used herein, the term "about" means quantity, level, value, number, frequency, percentage, dimension, size, amount, weight, or length that varies by 30%, 25%, 20%, 15%, 10%, 9%, 8%, 7%, 6%, 5%, 4%, 3%, 2%, or 0% based on reference quantity, level, value, number, frequency, percentage, dimension, size, amount, weight, or length.

### Nucleic Acid Sequence Notation

The symbols "A, T, C, G, and U" used herein are interpreted as meanings understood by those skilled in the art. Depending on the context and technique, the symbols may be appropriately interpreted as a base, nucleoside, or nucleotide on DNA or RNA. For example, when the symbols refer to bases, the symbols may be interpreted as adenine (A), thymine (T), cytosine (C), guanine (G), or uracil (U) themselves, respectively. When the symbols refer to nucleosides, the symbols may be interpreted as adenosine (A), thymidine (T), cytidine (C), guanosine (G), or uridine (U), respectively. When the symbols refer to nucleotides in a sequence, the symbols are required to be interpreted to mean nucleotides containing each of the nucleosides.

### Limitations of Conventional Art

The method for producing a recombinant adeno-associated virus may be divided into the following processes: a) an upstream process of producing a recombinant adeno-associated virus through host cells; and b) a downstream process of harvesting, filtering, separating, and purifying the recombinant adeno-associated virus produced in the host cells. The upstream process is directly related to a process by which the host cells express each component of the recombinant adeno-associated virus. The downstream process is directly related to a method of separating and purifying only the target recombinant adeno-associated virus from a mixture of various biological materials such as proteins, nucleic acids, and lipids.

Research on conventional recombinant adeno-associated virus production methods has limitations in that the methods focus only on improving the upstream process or downstream process, especially focusing on very few process improvements in the processes. Currently, there is very little research on how to design an entire method for producing a recombinant adeno-associated virus, including the upstream and downstream processes, to acquire high-quality recombinant adeno-associated virus particles with high efficiency.

### Method for Producing Recombinant Adeno-Associated Virus (rAAV)

### Overview of Method for Producing rAAV

The method for producing a recombinant adeno-associated virus (rAAV) disclosed herein includes preparing host cells; treating the host cells in pre-culture; culturing the host cells; offering an osmotic shock; and acquiring recombinant adeno-associated virus produced from the host cells and/or media containing the same. The method for producing a recombinant adeno-associated virus may be divided into the following processes: a) an upstream process of producing an adeno-associated virus through host cells and b) a downstream process of separating and purifying the adeno-associated virus produced in the host cells. In the upstream process, the method for producing a recombinant adeno-associated virus allows host cells to produce a recombinant adeno-associated virus in a more efficient way and higher production volume through the pre-culture treatment. In addition, the production method allows most of the recombinant adeno-associated virus produced by the host cells to be released into the media through the osmotic shock process, thereby omitting a process of cell disruption in the downstream process. This allows recovery of the recombinant adeno-associated virus in higher quality. The method for producing the recombinant adeno-associated virus includes the pre-culture treatment process in the upstream process and employs the osmotic shock process immediately before the downstream process. Thereby, the method allows the processes to create a synergistic effect, and as a result maximizes yield and quality.

Hereinafter, the method for producing the recombinant adeno-associated virus will be described in more detail.

### Host Cell Preparation Process

The method for producing a recombinant adeno-associated virus disclosed herein includes a host cell preparation process. The host cell preparation process refers to a process of preparing host cells capable of producing the recombinant adeno-associated virus to be produced.

The host cell preparation process is not otherwise limited as long as the host cells capable of producing the recombinant adeno-associated virus to be produced are prepared. The process of preparing the host cells may be performed by appropriately selecting a method known to those skilled in the art and modifying it to suit the method disclosed herein. For example, the host cell preparation process may use a method known in (paper).

An example of the host cell preparation process is exemplified in «Host Cell Preparation Process» in «Possible Embodiments of Invention».

### Pre-culture Treatment Process

The method for producing recombinant adeno-associated virus disclosed herein includes a pre-culture treatment process. The pre-culture treatment process refers to a process that allows the host cells to produce the recombinant adeno-associated virus more efficiently before culturing the host cells to produce the recombinant adeno-associated virus.

The pre-culture treatment process includes adding a pre-culture agent. The pre-culture agent contains sugar, Nocodazole, and/or M344.

The pre-culture treatment process disclosed herein serves to increase the yield of the recombinant adeno-associated virus instead of stopping a cell cycle and interfering with cell growth. As a result of the pre-culture treatment, the host cells are capable of producing more recombinant adeno-associated virus in culture. More specific details about the pre-culture treatment are described in a paragraph of «Pre-culture Process».

### Host Cell Culture Process

The method for producing a recombinant adeno-associated virus disclosed herein includes a host cell culture process. The host cell culture process refers to a process of appropriately treating host cells and/or media containing the host cells so that the host cells, for which the pre-culture treatment has been completed, may produce the recombinant adeno-associated virus. The term "appropriately treat" includes meanings such as adding specific substances to the host cells (e.g., adding nutrient medium), placing the host cells in a predetermined environment for a predetermined period of time (e.g., culturing the host cells in an incubator at room temperature for a predetermined period of time), changing an environment of the host cells to a new environment (e.g., replacing medium for subculture), or using a predetermined device to create a predetermined impact (e.g., using a shaker to vibrate the host cells and media). The term encompasses all technical ideas that those skilled in the art may recognize.

The host cell culture process is not otherwise limited as long as the host cells are capable of producing the recombinant adeno-associated virus. The process of culturing the host cells may be performed by appropriately selecting a method known to those skilled in the art and modifying it to suit the method disclosed herein.

### Osmotic Shock Process

The method for producing a recombinant adeno-associated virus disclosed herein includes an osmotic shock process. The osmotic shock process refers to a process that causes the host cells to release the recombinant adeno-associated virus into the media by applying an osmotic shock to the host cells producing and containing the recombinant adeno-associated virus within the cells.

By allowing the recombinant adeno-associated virus to be released into the media, the method for producing a recombinant adeno-associated virus may purify and/or acquire the recombinant adeno-associated virus without cell disruption. When acquiring the recombinant adeno-associated virus without cell disruption, the acquisition process is simplified, recombinant adeno-associated virus particles lost during the acquisition process are minimized, and intact recombinant adeno-associated virus particles may be recovered. As a result, the osmotic shock process contributes to increasing the quality of the recombinant adeno-virus produced by the method for producing a recombinant adeno-associated virus. More specific details about the osmotic shock process are described in a paragraph of «Osmotic Shock Process».

### Process of Acquiring Recombinant Adeno-associated Virus

The method for producing a recombinant adeno-associated virus disclosed herein includes a process of acquiring a recombinant adeno-associated virus. The process of acquiring a recombinant adeno-associated virus refers to a process of acquiring a recombinant adeno-associated virus produced from the host cells and/or media containing the host cells. The process of acquiring a recombinant adeno-associated virus is performed without cell disruption due to the presence of the osmotic shock process.

The process for acquiring a recombinant adeno-associated virus is not otherwise limited as long as the recombinant adeno-associated virus may be acquired. The process for acquiring the recombinant adeno-associated virus may be performed by appropriately selecting a method known to those skilled in the art and modifying it to suit the method disclosed herein.

In one embodiment, the process of acquiring a recombinant adeno-associated virus may be acquiring a recombinant adeno-associated virus from the host cells and/or media containing the host cells by a chromatographic method. Specifically, the chromatographic method may be a method disclosed in Rieser R, Koch J, Faccioli G, Richter K, Menzen T, Biel M, Winter G, Michalakis S. Comparison of Different Liquid Chromatography-Based Purification Strategies for Adeno-Associated Virus Vectors. Pharmaceutics. 2021; 13(5):748. Https:/doi.org/10.3390/pharmaceutics13050748, or an appropriate modification thereof.

In another embodiment, the process of acquiring a recombinant adeno-associated virus may be acquiring a recombinant adeno-associated virus from the host cells and/or media containing the host cells by an ultracentrifugation method. Specifically, the ultracentrifugation method may be a method disclosed in Sena-Esteves M, Gao G. Purification of Recombinant Adeno-Associated Viruses (rAAVs) by Iodixanol Gradient Centrifugation. Cold Spring Harb Protoc. 2020 Feb 3;2020(2):095612. doi: 10.1101/pdb.prot095612. PMID: 32015002., or an appropriate modification thereof.

### Characteristics of Method for Producing Recombinant Adeno-associated Virus

### Overview of Characteristics of Method for Producing Recombinant Adeno-associated Virus

The method for producing a recombinant adeno-associated virus disclosed herein includes a pre-culture treatment process and an osmotic shock process. By including the processes, the method may produce a recombinant adeno-associated virus in high quality and high yield.

To compare the characteristics of various recombinant adeno-associated virus production methods and select a superior production method, criteria are needed to compare the yield and quality of the recombinant adeno-associated viruses produced. Herein, it is inappropriate to compare the yield and quality using only one criterion, and it is essential to compare the yield and quality using various criteria.

In this specification, various criteria for evaluating a recombinant adeno-associated virus produced by various production methods are intended to disclose. Furthermore, based on this, inventor(s) would like to reveal that the production method disclosed herein is significantly superior in many aspects to conventionally disclosed production methods.

### Evaluation Method for Production Method 1 - Quantity and Yield of rAAV Produced

As a criterion for evaluating the quality of the produced and acquired recombinant adeno-associated virus, production or yield is disclosed herein. This is an indicator of how much the recombinant adeno-associated virus is produced when using the same amount of host cells, that is, cells producing the recombinant adeno-associated virus. To mass-produce the recombinant adeno-associated virus, it is basically efficient to select a production method that produces a large number of virus particles (or vectors) per host cell. Therefore, the production, or yield, of recombinant adeno-associated virus is a basic quantitative indicator for comparing the superiority and inferiority between production methods.

In one embodiment, the production amount of recombinant adeno-associated virus may be determined by measuring the number of total viral genomes (vg) in the measurement target. In another embodiment, the yield of the recombinant adeno-associated virus by each production method may be compared by measuring and comparing the number of viral genomes produced in the same amount of host cells.

### Evaluation Method for Production Method 2 - Full Capsid-empty Capsid Ratio

As a criterion for evaluating the quality of the produced and acquired recombinant adeno-associated virus, a full capsid-empty capsid ratio is disclosed herein. Herein, the full capsid refers to a recombinant adeno-associated virus particle in which a genetic material is properly packaged within a capsid. On the contrary, an empty capsid refers to a particle in which a genetic material is not properly packaged within a capsid. The empty capsid may be toxic when administered to a living organism. Therefore, removing the empty capsid during the production process and increasing the ratio of the full capsid is a very important task when using the recombinant adeno-associated virus as a therapeutic agent. The higher the ratio of the full capsid to the empty capsid, the higher the quality of the recombinant adeno-associated virus produced.

In one embodiment, the ratio of full capsid to empty capsid may be acquired by performing negative staining on the produced recombinant adeno-associated virus samples and then taking and analyzing TEM photographs.

### Evaluation Method for Production Method 3 - Genome Integrity

As a criterion for evaluating the quality of the produced and acquired recombinant adeno-associated virus, a genome integrity ratio is disclosed herein. Herein, the genome integrity refers to measuring a proportion of particles in which an "intended genetic material" in a capsid is properly packaged by performing Digital-PCR on the produced recombinant adeno-associated virus. This measures a percentage at which the recombinant adeno-associated virus is produced as designed. This serves as a criterion for measuring the quality of the produced recombinant adeno-associated virus. Compared to the full capsid-empty capsid ratio, it is a method of measuring the quality of virus particles acquired from different perspectives, so the method may be used as a complementary quality criterion.

In one embodiment, the genomic integrity of the produced recombinant adeno-associated virus is measured by PCR to determine whether ITR and bGHpA target genes are included in a transgene of the recombinant adeno-associated virus in each sample. Therefore, the genomic integrity may be measured as a ratio of the number of viruses containing both the ITR and bGHpA target genes to the total number of recombinant adeno-associated virus produced.

### Evaluation Method for Production Method 4 - Purity

As a criterion for evaluating the quality of the produced and acquired recombinant adeno-associated virus, purity of a final product acquired is disclosed herein. Herein, the purity refers to a measure of how purely a final product acquired contains only recombinant adeno-associated virus. For example, when the final product acquired contains a lot of cell byproducts (proteins and nucleic acids) other than the recombinant adeno-associated virus, the purity is low. When the final product acquired contains only recombinant adeno-associated virus, the purity is significantly high. In one embodiment, the purity may be measured by checking how much of a background band appears through silver staining and Coomassie brilliant blue staining. Generally, a substance to be used as a therapeutic agent is the recombinant adeno-associated virus itself. Under the condition that it is unclear that impurity presence may cause side effects or toxicity, the high purity of the final product is also an important criterion.

### Characteristics of Method for Producing Recombinant Adeno-associated Virus

The method for producing a recombinant adeno-associated virus disclosed herein includes 1) a pre-culture treatment process and 2) an osmotic shock process in terms of configuration, so that a downstream process may proceed without cell disruption. Due to these characteristics, the production method disclosed herein may 1) produce a recombinant adeno-associated virus in high yields, 2) exhibit a high full capsid-empty capsid ratio, 3) exhibit a high percentage of genomic integrity, and 4) have a high purity of the final product, as compared to the conventional production methods that do not include the configuration. Furthermore, the production method disclosed herein increases production and quality of recombinant adeno-associated virus particles, reduces time and cost required for the purification process, and enables more economical production of the recombinant adeno-associated virus. In conclusion, using the recombinant adeno-associated virus production method disclosed herein, it is possible to produce the recombinant adeno-associated virus of the intended composition in significantly higher yield and significantly higher quality than conventional production methods.

### Host Cell Preparation Process

The method for producing a recombinant adeno-associated virus disclosed herein includes a host cell preparation process. Host cells are capable of producing the recombinant adeno-associated virus. To be able to produce the recombinant adeno-associated virus, the host cells contain: 1) a nucleic acid encoding an envelope (capsid) of the recombinant adeno-associated virus; and 2) a nucleic acid encoding a predetermined genetic material to be included in the envelope. Furthermore, the host cells may further contain a nucleic acid that encodes various substances and information 3) to ensure that the envelope and the genetic material may be expressed within the host cells, and further to ensure that the genetic material is well contained (packaged) within the expressed envelope. The host cells may contain the nucleic acids of 1) to 3) in a well-known vector form (e.g., plasmid). To prepare the host cells, a process of transfecting the nucleic acids of the 1) to 3) into a predetermined cell line may be necessary. As mentioned above, the process of preparing host cells may be performed by appropriately selecting a method known to those skilled in the art and modifying it to suit the method disclosed herein. The host cells may produce the recombinant adeno-associated virus when an appropriate environment is provided. In the process described below, cells referred to as "host cells" refer to cells capable of producing the recombinant adeno-associated virus.

### Pre-culture Treatment Process

### Overview of Pre-culture Treatment Process

The method for producing a recombinant adeno-associated virus disclosed herein includes a pre-culture treatment process. The pre-culture treatment process allows host cells to produce recombinant an adeno-associated virus more efficiently by treating the host cells with a pre-culture agent containing one or more selected from the group consisting of sugar, nocodazole, and M344. Specifically, 1) the sugar serves to increase the amount of recombinant protein (herein, recombinant adeno-associated virus) produced by the host cells. 2) The nocodazole serves to increase the production of recombinant adeno-associated virus by fixing the host cells in a specific cell cycle. 3) The M344 serves to increase the production of the virus by inhibiting the decomposition mechanism of the recombinant adeno-associated virus envelope protein. This allows the host cells to produce the recombinant adeno-associated virus more efficiently and in greater quantities.

### Sugar

The pre-culture treatment process may include adding sugar to the host cells. The addition of the sugar may mean treating the host cells with a pre-culture agent containing the sugar. The sugar is not limited as long as the sugar may be used as a nutrient within the cell. For example, the sugar may be sucrose or sorbitol, but is not limited thereto. When treating the host cells with the sugar, the amount of recombinant protein (here, recombinant adeno-associated virus) produced by the host cells may be increased. The sugar does not affect the downstream process of the recombinant adeno-virus production process, and only affects the process by which the host cell produces the recombinant adeno-associated virus.

### Nocodazole

The pre-culture treatment process may include adding nocodazole to the host cells. The addition of the nocodazole may mean treating the host cells with a pre-culture agent containing the nocodazole. The nocodazole is an antimitotic agent. It is known that nocodazole acts on cells to interfere with cell division and proliferation by interfering with segregation of chromosomes. When treating the host cells with nocodazole, the host cells are fixed in a G2/M cell cycle. Thereby, when compared to cells not treated with the nocodazole, the host cells experience an increase in an average cell volume and express more recombinant proteins (i.e., recombinant adeno-associated virus particles) on average.

### M344

The pre-culture treatment process may include adding M344 to the host cells. The addition of the M344 may mean treating the host cells with a pre-culture agent containing the M344. The M344 is a histone deacetylase inhibitor. The M344 may inhibit deacetylation of histones within cells and the degradation of ubiquitinated proteins, including viral capsid proteins. When the M344 is added to the host cells, the degradation of the recombinant adeno-associated virus protein in the host cells is inhibited and more virus particles are expressed.

### Osmotic Shock Process

### Overview of Osmotic Shock Process

The method for producing a recombinant adeno-associated virus disclosed herein includes an osmotic shock process. The osmotic shock process refers to a process of maintaining osmolarity outside the host cells higher than the osmolarity inside the host cells for a predetermined period of time. As long as the objective is achieved, the "method for increasing osmolarity" is not otherwise limited. For example, the osmotic shock process may include adding an osmotic shock reagent containing a salt to the host cells. Additionally, additional substances may be added to stabilize the recombinant adeno-associated virus released during the osmotic shock process. For example, the additional material may be, but is not limited to, a non-ionic surfactant.

### Salt

The osmotic shock process includes adding an osmotic shock reagent containing a salt to the host cells. Herein, the salt is used to maintain a high osmolality outside the host cells (i.e., in the media), and is not otherwise limited as long as the salt is not toxic to the host cells. For example, the salt may be a salt containing a monovalent ion such as sodium chloride (NaCl) or potassium chloride (KCl). For another example, the salt may be a salt containing a divalent ion such as magnesium chloride (MgCl₂).

### Non-ionic Surfactant

The osmotic shock reagent used in the osmotic shock process may further comprise a non-ionic surfactant.. Herein, the non-ionic surfactant stabilizes the recombinant adeno-associated virus particles released by the host cells into the media, thereby allowing more recombinant adeno-associated virus particles to be recovered from the media. For example, the non-ionic surfactant may be Plucuronic acid (PF68), but is not limited thereto.

### [Possible Examples of Invention]

### Host Cell Preparation Process

### Example 1, Host Cell Preparation Process

A process of preparing, bringing, and/or providing host cells.

### Example 2, Media Inclusion

In Example 1, the process of preparing, bringing, and/or providing the host cells means a process of preparing, bringing, and/or providing the host cells and media containing the host cells.

### Example 3, rAAV Expression Possibility

In any one of Examples 1 to 2, the host cells are capable of expressing a recombinant adeno-associated virus (rAAV).

### Example 4, rAAV Vector Inclusion

In Example 3, the host cells contain a rAAV expression vector.

### Example 5, Limit to serotype of rAAV

In any one of Examples 3 to 4, the rAAV is a serotype selected from one or more of the following:
wild-type serotypes including AAV1, AAV2, AAV3, AAV4, AAV5, AAV6, AAV7, AAV8, AAV9, and AAV10;
synthetic (variant) serotypes including AAV-DJ, AAV-DJ8, AAV-DJ9, and AAV6.2; and
other AAV variant serotypes.

### Example 6, Limit to Host Cells

In any one of Examples 1 to 5, the host cells are one or more selected from the following:
HEK293T; and Expi293F.

### Pre-culture Process

### Example 7, Pre-culture Process

A process of adding, mixing, treating, and/or introducing a pre-culture agent to host cells.

### Example 8, Addition of Pre-culture Agent to Media

In Example 7, the process of adding, mixing, treating, and/or introducing a pre-culture agent to the host cells means a process of adding, mixing, treating, and/or introducing a pre-culture agent into the media containing the host cells.

### Example 9, Pre-culture Agent Specification

In Example 7, the pre-culture agent includes one or more selected from the following:
sugar; nocodazole; and M344.

### Example 10, Pre-culture Process Time Specification

In any one of Examples 7 to 9, the pre-culture agent is applied at a predetermined time point after a previous process (e.g., a host cell preparation process),
herein, the predetermined time point is selected from the following:
a time point selected in real numbers between 0 and 1440, representing a time point at an arbitrary number of minutes has elapsed, for example, 30 minutes after (at);
a time point selected in real numbers between 0 and 1440, representing a time point after an arbitrary number of minutes have elapsed, for example, 60 minutes after (after);
a time point selected in real numbers between 0 and 1440, representing a time point before an arbitrary number of minutes have elapsed, for example, 90 minutes after (before);
a time point selected in real numbers between 0 and 1440, representing a time point within two arbitrary numerical ranges, for example, after 180 minutes before 300 minutes (after A and before B); or
a time point before 30 minutes, about 30 minutes, about 1 hour, about 1 hour 30 minutes, about 2 hours, of about 2 hours 30 minutes, about 3 hours, about 3 hours 30 minutes, about 4 hours, about 4 hours 30 minutes, or about 5 hours later.

### Example 11, Conceptualization of Yield Improvement Process

A process for increasing production of a recombinant adeno-associated virus, including any one of Examples 7 to 10.

### Osmotic Shock Process

### Example 12, Osmotic Shock Process

A process of adding, mixing, treating, and/or introducing an osmotic shock reagent to host cells.

### Example 13, Addition of Osmotic Shock Reagent to Media

In Example 12, the process of adding, mixing, treating, and/or introducing an osmotic shock reagent to host cells means a process of adding, mixing, treating, and/or introducing an osmotic shock reagent into the media containing the host cells.

### Example 14, Osmotic Shock Reagent Specification

In any one of Examples 12 to 13, the osmotic shock reagent includes one or more selected from the following:
a salt; and
a non-ionic surfactant.

### Example 15, Limit to Salt Type

In Example 14, the salt includes one or more selected from the following:
sodium chloride (NaCl); potassium chloride (KCl); and magnesium chloride (MgCl₂).

### Example 16, Limit to Salt Concentration

In any one of Examples 14 to 15, the salt concentration is one or more selected from the following:
a concentration selected from about 0.1 M, about 0.2 M, about 0.3 M, about 0.4 M, about 0.5 M, about 0.6 M, about 0.7 M, about 0.8 M, about 0.9 M, about 1.0 M, about 1.1 M, about 1.2 M , about 1.3 M, about 1.4 M, about 1.5 M, about 1.6 M, about 1.7 M, about 1.8 M, about 1.9 M, or about 2.0 M;
a concentration within a range consisting of two values selected from the group consisting of about 0.1 M, about 0.2 M, about 0.3 M, about 0.4 M, about 0.5 M, about 0.6 M, about 0.7 M, about 0.8 M, about 0.9 M, about 1.0 M, about 1.1 M, about 1.2 M, about 1.3 M, about 1.4 M, about 1.5 M, about 1.6 M, about 1.7 M, about 1.8 M, about 1.9 M, and about 2.0 M, for example, about 0.1 M to about 1.5 M;
a concentration selected from about 0.1 M or less, about 0.2 M or less, about 0.3 M or less, about 0.4 M or less, about 0.5 M or less, about 0.6 M or less, about 0.7 M or less, about 0.8 M or less, about 0.9 M or less, about 1.0 M or less, about 1.1 M or less, about 1.2 M or less, about 1.3 M or less, about 1.4 M or less, about 1.5 M or less, about 1.6 M or less, about 1.7 M or less, about 1.8 M or less, about 1.9 M or less, or about 2.0 M or less; and
a concentration selected from about 0.1 M or more, about 0.2 M or more, about 0.3 M or more, about 0.4 M or more, about 0.5 M or more, about 0.6 M or more, about 0.7 M or more, about 0.8 M or more, about 0.9 M or more, about 1.0 M or more, about 1.1 M or more, about 1.2 M or more, about 1.3 M or more, about 1.4 M or more, about 1.5 M or more, about 1.6 M or more, about 1.7 M or more, about 1.8 M or more, about 1.9 M or more, or about 2.0 M or more.

### Example 17, Limit to Non-ionic Surfactant

In any one of Examples 14 to 16, the non-ionic surfactant is PF68.

### Example 18, Osmotic Shock Time Specification

In any one of Examples 12 to 17, the process is performed for a predetermined time,
herein, the predetermined time is selected from the following:
a time selected in real numbers between 0 and 1440, representing a time at an arbitrary number of minutes have elapsed, for example, 30 minutes;
a time selected in real numbers between 0 and 1440, representing a time after an arbitrary number of minutes have elapsed, for example, 60 minutes or more;
a time selected in real numbers between 0 and 1440, representing a time before an arbitrary number of minutes have elapsed, for example, 90 minutes or less; or
a time of about 30 minutes or less, about 30 minutes, about 1 hour, about 1 hour and 30 minutes, about 2 hours, about 2 hours and 30 minutes, about 3 hours, about 3 hours and 30 minutes, about 4 hours, about 4 hours and 30 minutes, or about 5 hours.

### Example 19, Limit to Media Osmolarity

In any one of Examples 12 to 18, due to this process, osmolarity outside the host cells is maintained at a concentration selected from:

### Example 20, Conceptualization of Quality Improvement Process

A process for improving the quality of a recombinant adeno-associated virus, including any one of Examples 12 to 19.

### Example 21, Implications for Quality Improvement

In Example 20, the improving the quality of the recombinant adeno-associated virus means any one selected from the following:
an increase in a full capsid-empty capsid ratio of the acquired recombinant-adeno associated virus;
an increase in purity of the acquired recombinant-adeno associated virus, or a reduction in cell debris contained along with acquired recombinant-adeno associated virus; and
an increase in genome integrity of the acquired recombinant-adeno-associated virus.

### rAAV Acquisition Process

### Example 22, rAAV Acquisition Process

A process of acquiring, extracting, and/or purifying a recombinant adeno-associated virus (rAAV) without disrupting, lysing, and/or destructing the host cells.

### Example 23, Chromatography Process

In Example 22, the rAAV acquisition process further includes:
(e-1) separating a medium from the host cells in a resulting product of a previous process;
(e-2) filtering the medium;
(e-3) performing affinity chromatography on the resulting product of the step (e-2); and
(e-4) performing anion exchange chromatography on the resulting product of the step (e-3).

### Example 24, Ultracentrifugation Process

In Example 22, the rAAV acquisition process further includes:
(e-1) separating the medium from the host cells in the product of step (d);
(e-2) filtering the medium;
(e-3) performing a PET precipitation on the product of step (e-2);
(e-4) adding benzonase to the product of step (e-3) in the presence of a salt;
(e-5) performing a 1st iodixanol gradient ultracentrifugation on the product of step (e-4); and
(e-6) performing a 2nd iodixanol gradient ultracentrifugation on the product of step (e-5).

### rAAV Production Method

### Example 25, rAAV Production Method

A method for producing a recombinant adeno-associated virus (rAAV) including:
(a) performing a host cell preparation process of any one of Examples 1 to 6;
(b) performing a pre-culture process of any one of Examples 7 to 11 on host cells prepared in the step (a);
(c) performing a process of culturing, incubating, proliferating, and/or growing the host cells that have completed the step (b);
(d) performing an osmotic shock process of any one of Examples 12 to 21 on the host cells that have completed the step (c); and
(e) performing a recombinant adeno-associated virus acquisition process of any one of Examples 22 to 24 on the host cells that have completed the step (d).

### [Experimental Examples]

Hereinafter, the present disclosure provided by this specification will be described in more detail through experimental examples and examples. These examples are intended solely to illustrate the content disclosed by this specification. It will be apparent to those skilled in the art that the scope of the content disclosed by this specification is not to be construed as limited by these examples.

### Experimental Example 1. Experimental methods and materials

### Experimental Example 1.1. Preparation of Recombinant Adeno-associated Virus Expression Plasmids

Among three recombinant adeno-associated virus plasmids - Addgene #202364 (pAAVCMV.PI.eGFP.WPRE.bGH), Addgene #327732 pRepCap9, and Addgene #244764 pHelper, pAAV was transformed into NEB Stable competent cell strain (CAT.C3040H), while the other two plasmids were transformed into Dyne bio DH5a strain (DYO1350). A culture method involved subculturing in 3 L units, followed by cryopreservation of E. coli cells transformed with each plasmid to create frozen cell stocks. These frozen cells were then subcultured in large volume (Culture medium: LB broth (BD DIFCO 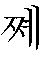 244620)). By using a plasmid extraction kit(Nucleobond PC 10000 EF, Giga kit for endotoxin-free plasmid DNA(Macherey-Nagel, CAT.740548)), plasmids were prepared.

### Experimental Example 1.2. Host Cell Preparation

Expi293F cells (Cat#A14527, Thermo) were grown in Expi293 Expression Medium (Cat# A14351-02, Thermo) supplemented with 1% penicillin-streptomycin (Cat#15140122, Thermo). Then, the cells were maintained under serum-free suspension culture conditions. Herein, the serum-free suspension culture conditions were as follows: 120 rpm stirring rate in a shaker incubator (Cat#NB-206CL, N-biotek, South Korea); 37°C; 8% CO₂; and 85% relative humidity.

To produce recombinant adeno-associated viral vectors on a large scale, the cells were cultured in 250 mL medium in a 1 L Erlenmeyer flask (Cat#784011, Nest) with agitation at 120 rpm.

### Experimental Example 1.3. Transfection of Recombinant Adeno-associated Virus Expression Plasmid into Host Cells

Expi293F cells were seeded in Expi293F expression media at a density of 2.5 x 10^6 cells per 1 mL and cultured for 3 hours. Then, the cells were transfected using 25-kDa linear PEI (Cat#23966-1, Polyscience, USA) along with the three plasmids (pHelper[Cat#112867, Addgene], pRC9n[Cat#112865, Addgene], and pAAV[Cat#105530, Addgene]), respectively. Specifically, the plasmids were mixed in DMEM (Cat#10569010, Thermo) at 1/10 the volume of cells to be transfected. Next, PEI was added to DNAs diluted in DMEM. After an additionally 20-minute culture, a DNA-PEI complex was added to the cells.

### Experimental Example 1.4. Harvest before Recombinant Adeno-Associated Virus Purification 1 - Osmotic Shock

96 hours after the transfection according to Experimental Example 1.3, an osmotic shock reagent (using 0.5 M NaCl aqueous solution) was added to the host cells, and the condition was maintained for 3 hours. Then, a host cell culture was collected into a 1000 mL polypropylene conical tube by being poured into a shaking flask. Next, the cell culture was centrifuged at 3300 X g for 30 minutes at 4°C using a highspeed centrifuge (Cat#SU-R22, Hanil) and Angle Rotor (Cat#A1000-4. Hanil). The cells were discarded, and the supernatant was used for purification using a filtrate 0.45 PES filter system.

### Experimental Example 1.5. Harvest before Recombinant Adeno-Associated Virus Purification 2 - Freezing and Thawing

96 hours after the transfection according to Experimental Example 1.3, the cells were frozen and thawed for 6 cycles or more. Herein, liquid nitrogen at -197°C was used to freeze the cells, and the cells were thawed in a water bath at 37°C. Then, the recombinant adeno-associated virus particles in each medium was precipitated in 1/4 cell culture volume of 40% PEG 8000 (Cat# V3011, Promega), 2.5 M NaCl, and pH 7.4. Next, each precipitate was cultured at 4°C overnight, and the recombinant adeno-associated virus in each medium was precipitated by centrifugation at 3,300 x g for 20 minutes. The precipitate was resuspended in resuspension buffer (1xPBS, 250 mM NaCl, 0.001% PF-68, and pH 7.5). After a Benzonase treatment (50 U/mL for 1 hour at 37°C), cell debris was spun down at 4,000 rpm for 30 minutes.

### Experimental Example 1.6. Recombinant Adeno-Associated Virus Purification - Ultracentrifugation Method

For the recombinant adeno-associated virus harvested through Experimental Example 1.4 or Experimental Example 1.5, an ultracentrifugation process was performed using the following method to purify the recombinant adeno-associated virus:
1) The recombinant adeno-associated virus was pipetted into a Beckman Quick Seal tube using a pipetting needle attached to a 10 mL syringe.
2) 6 mL of 15% iodixanol (Cat#PB-1893, Progen), 6 mL of 25% iodixanol, 6 mL of 40% iodixanol, and 5 mL of 60% iodixanol were sequentially layered from the bottom of a Beckman Quick Seal tube into which the recombinant adeno-associated virus was pipetted.
3) Then, 1X PBS was added to completely fill the Beckman Quick Seal tube.
4) The Beckman Quick Seal tube of the step 3) was centrifuged at 350,000 x g for 1.5 hours at 10°C using a 70Ti rotor.
5) Next, to recover recombinant adeno-associated virus particles, the Beckman Quick Seal tube was fixed to a clamp stand set at eye level, and an 18G needle was inserted into the top of the tube to allow air to enter.
6) Afterward, another 18G needle was inserted just below the interface of the 40% and 60% iodixanol layers in the tube with the needle's inclination facing upward.
7) Using the 18G needle, about 4 mL of the recombinant adeno-associated virus samples contained in the 40% iodixanol layer were extracted.
8) The extracted samples were centrifuged again at 350,000 x g for 1.5 hours at 10°C using the 70Ti rotor.
9) Afterward, processes of the steps 5) to 7) were repeated again to acquire purified a recombinant adeno-associated virus.

### Experimental Example 1.7. Concentration and Buffer Exchange

First, Viva Spin 20 concentrates were coated with 0.1%, 0.01%, and 0.001% PF-68, respectively. Then, the recombinant adeno-associated virus samples extracted according to Experimental Example 1.6 were added to 10 mL of preparation buffer solution (1xPBS, 220 mM NaCl, 5% D-sorbitol, and pH 7.5) pre-distributed at the upper part of a Vivaspin 20 100,000MWCO (cat#VS2020, Sartorius) concentrator and centrifuged at 3000 x g for 10 minutes, respectively. After most of the solution sufficiently passed through a filter, 10 mL of PBS was added, and washing and spinning were repeated 5 times. After the final washing, a formulation buffer solution was rotated repeatedly until 200 µL remained, and then the virus in each sample was aliquoted and stored at -80°C for future use.

### Experimental Example 1.8. Silver Staining

For use in SDS-PAGE gels, the recombinant adeno-associated virus samples were prepared by adding an appropriate amount of NuPAGE lithium dodecyl sulfate (LDS) sample buffer (Cat#NP0007, Thermo). Then, the samples were placed at 70°C for 10 minutes. Next, the same amount of recombinant adeno-associated virus in each sample was loaded onto a Bolt 4-12% Bis-Tris gel (Cat# NW04122BOX, Thermo), and then was subjected to electrophoresis in Bolt 2-(N-morpholino)esulfonic acid (MES) SDS Running Buffer (Cat#B0002, Thermo). After the electrophoresis, the gel was stained using a SilverXpress silver staining kit (Cat#LC6100, Thermo) according to the manufacturer's instructions.

### Experimental Example 1.9. Coomassie Brilliant Blue Staining

Before performing Coomassie brilliant blue staining, the recombinant adeno-associated virus samples were cultured at 95°C for 10 minutes. Then, 200 V ran through a gel for 40 minutes until a loading dye reached the bottom of the gel. The gel was soaked in distilled water and cultured twice for 5 minutes each. Next, the gel was placed in a Coomassie Brilliant Blue R-250 staining solution (Cat#EBC001-1000, Enzynomics) and cultured for 1 hour with gentle agitation. Afterward, the gel was decolorized with a decolorizing solution (80% distilled water, 10% methanol, and 10% acetic acid). Afterward, the decolorizing solution was removed and decolorized with a new decolorizing solution.

### Experimental Example 1.10. qRT-PCR

To perform qRT-PCR, Dnase I was added to the samples, and the samples were cultured at 37°C for 30 minutes. Then, Proteinase K was added, and the samples were cultured at 55°C for 30 minutes. Next, the samples were inactivated by standing at 95°C for 15 minutes.

Herein, a pTR UF-11 plasmid (ATCC MBA-331) was used as a standard plasmid, and a reference standard stock AAV8 (ATCC VR-1816) was used as a positive control.

The primers used in the qRT-PCR were as follows:
1) ITR (Forward: GGAACCCCTAGTGATGGAGTT(sequence ID number 1), Reverse: CGGCCTCAGTGAGCGA(sequence ID number 2), Prv: CACTCCCTCTCTGCGCGCTCG(sequence ID number 3))
2) bGH (Forward: GCCAGCCATCTGTTGT(sequence ID number 4), Reverse: GGAGTGGCACCTTCCA(sequence ID number 5), Prv: TCCCCCGTGCCTTCCTTGACC(sequence ID number 6))

The running protocol used was 10 minutes at 95°C, followed by 40 cycles of (30 seconds at 95°C and 1 minute at 60°C) conditions. After the processes were completed, the samples were analyzed with QuantStudio3 (Applied Biosystem).

### Experimental Example 1.11. Digital PCR

To perform Digital PCR, Dnase I was added to the samples, and the samples were cultured at 37°C for 30 minutes. Afterward, Proteinase K was added, and the samples were cultured at 55°C for 30 minutes. Next, the samples were inactivated by leaving them as they were at 95°C for 15 minutes. Afterward, the samples were serially diluted from 10⁵ to 10⁷.

The primers used in the Digital-PCR were as follows:
1) ITR (Forward: GGAACCCCTAGTGATGGAGTT(sequence ID number 1), Reverse: CGGCCTCAGTGAGCGA(sequence ID number 2), Prv: CACTCCCTCTCTGCGCGCTCG(sequence ID number 3))
2) bGH (Forward: GCCAGCCATCTGTTGT(sequence ID number 4), Reverse: GGAGTGGCACCTTCCA(sequence ID number 5), Prv: TCCCCCGTGCCTTCCTTGACC(sequence ID number 6))

The following processes were used as a running protocol: 10 minutes at 95°C; 40 cycles of 30 seconds at 94°C and 1 minute at 60°C; and 10 minutes at 98°C. After the processes were completed, analyses were performed using the QIACuity dPCR system (Qiagen).

### Experimental Example 1.12. TEM Analysis

Analyses were conducted using TEM to visualize the recombinant adeno-associated virus particles. Negative staining analysis was performed to analyze the full capsid-empty capsid ratio. Specifically, 1) 10 µL of samples were placed in formvar/carbon coated Ni. grid for 5 minutes, 2) The samples were stained with 5% uranyl acetate for 2 minutes. 3) After washing a dye used, the samples were dried in air for 30 minutes. 4) The samples were analyzed using Match Finder company (Tecnai G2 spirit twin (FEI, Netherlands, accelerating voltage 120kV).

### Experimental Example 2. Experiment on Yield Improvement Effect of Recombinant Adeno-associated Virus Production Process including Osmotic Shock Process

### Experimental Example 2.1. Experimental Conditions and Methods

The following experiment was conducted to determine a yield improvement effect of the recombinant adeno-associated virus production process including the osmotic shock process depending on osmotic shock conditions:
Host cells capable of producing recombinant adeno-associated virus of the AAV9 serotype were prepared according to Experimental Examples 1.1 to 1.3.

For each experimental group, 2.5 X 10⁶ cells per mL were seeded in a 30 mL culture vessel, and experiments were conducted using a total of 16 culture vessels for each experimental group.

On the basis of the time of transfection of the recombinant adeno-associated virus expression plasmid into the host cells, an osmotic shock reagent was added 4 days later, the condition was maintained for 3 hours, sample media and/or cells were harvested, and the viral genome was (vg) counts were measured.

### Experimental Example 2.2. Salt Addition, Yield Improvement Effect by Concentration

According to Experimental Example 2.1, the yield improvement effect of the osmotic shock process was measured for each salt concentration.

The composition of the osmotic shock reagent for each experimental group is shown in the following table.

**[Table 1]**

| Label | Media volume (mL) | Transfection (mL) | Salt shock (mL) | Total volume (mL) |
|---|---|---|---|---|
| NT | 30 | 3 | 0 | 33 |
| 0.1M NaCl | 30 | 3 | 0.66 | 33.66 |
| 0.2M NaCl | 30 | 3 | 1.32 | 34.32 |
| 0.5M NaCl | 30 | 3 | 3.3 | 36.3 |
| 1.0M NaCl | 30 | 3 | 6.6 | 39.6 |

Experimental results showed that as the salt concentration causing osmotic shock increased, the number of viral genomes recovered from the media increased (Figure 1). This showed that when the salt concentration increased to 1.0 M, the host cells actively released the recombinant adeno-associated virus particles into the media.

### Experimental Example 2.3. Salt Addition and Yield Improvement Effect by Salt Type

According to Experimental Example 2.1, the yield improvement effect of the osmotic shock process for each salt type was measured.

The composition of the osmotic shock reagent for each experimental group is shown in the following table.

**[Table 2]**

| Label | Media volume (mL) | Transfection (mL) | Salt shock (mL) | Total volume (mL) |
|---|---|---|---|---|
| NT | 30 | 3 | 0 | 33 |
| 0.5M NaCl | 30 | 3 | 3.3 | 36.6 |
| 0.5M KCl | 30 | 3 | 5.5 | 38.5 |
| 0.5M MgCl₂ | 30 | 3 | 16.5 | 39.5 |

Experimental results showed that NaCl, KCl, and MgCl₂ all showed a significant increase in the number of viral genomes recovered from the media compared to the negative control (NT) without osmotic shock (Figure 2). This could be interpreted as the host cells releasing recombinant adeno-associated virus particles into the media regardless of the salt type.

### Experimental Example 3. Experiment on Yield Improvement Effect of Recombinant Adeno-associated Virus Production Process including Pre-culture Treatment Process

### Experimental Example 3.1. Experimental Conditions and Methods

The following experiment was conducted to determine a yield improvement effect of the recombinant adeno-associated virus production process including the pre-culture treatment process depending on pre-culture treatment conditions:
Host cells capable of producing a recombinant adeno-associated virus of a AAV9 serotype were prepared according to Experimental Examples 1.1 to 1.3.

For each experimental group, 2.5 X 10⁶ cells per mL were seeded in a 30 mL culture vessel, and experiments were conducted using a total of 8 culture vessels for each experimental group.

On the basis of the time of transfection of the recombinant adeno-associated virus expression plasmid into the host cells, a pre-culture treatment process was performed 4 hours later, and after 4 days, the osmotic shock reagent was added, the condition was maintained for 3 hours, and sample media and/or cells were harvested to measure the number of viral genomes (vg). Herein, the pre-culture treatment process was performed by adding a pre-culture agent to the host cells.

### Experimental Example 3.2. Yield Improvement Effect when Performing Pre-culture Treatment with Nocodazole and M344

According to Experimental Example 3.1, the yield improvement effect was measured when the pre-culture treatment was performed with Nocodazole and/or M344.

The composition of a pre-culture treatment agent for each experimental group is shown in the following table:

**[Table 3]**

| Label | Pre-culture agent |
|---|---|
| Control | None |
| PEI (1mg/ml) | PEI (1mg/ml) |
| Nocodazole | PEI (1mg/ml), Nocodazole (4 uM) |
| M344 | PEI(1mg/ml), M344 (2.5 uM) |
| Nocodazole+M344 | PEI (1mg/ml), Nocodazole (4 uM), M344 (2.5 uM) |

Experimental results showed that when Nocodazole and M344 were added during the pre-culture treatment process, the yield of recombinant adeno-associated virus in the host cells was increased (Figure 3). What is noteworthy was that when Nocodazole and M344 were added simultaneously, a much higher yield was obtained than when Nocodazole and M344 were added individually during the pre-culture treatment process. In conclusion, when both Nocodazole and M344 were added in the pre-culture treatment process, it showed that Nocodazole and M344 created a synergy and had a significant impact on improving yield.

### Experimental Example 3.3. Yield Improvement Effect when Performing Pre-culture Treatment with Sugar, Nocodazole, and M344

According to Experimental Example 3.1, the yield improvement effect was measured when the pre-culture treatment was performed with Nocodazole, M344, and/or sugar.

The composition of a pre-culture treatment agent for each experimental group is shown in the following table:

**[Table 4]**

| Label | Pre-culture agent |
|---|---|
| Control | None |
| Sucrose | PEI, Sucrose(0.1 M) |
| Nocodazole+M344 | PEI, Nocodazole (4 uM), M344 (2.5 uM) |
| Sucrose+Nocodazole+M344 | PEI, Sucrose, Nocodazole (4 uM), M344 (2.5 uM) |

Experimental results showed that the yield improvement was higher when Nocodazole and M344 were added together than when only sugar (sucrose) was added during the pre-culture treatment process (FIG. 4).

### Experimental Example 4. Experiment on Yield and Quality Improvement Effect of Recombinant Adeno-associated Virus Production Method including Pre-culture Treatment and Osmotic Shock Process

### Experimental Example 4.1. Production methods to be compared

In this experimental example, the method for producing a recombinant adeno-associated virus, including pre-culture treatment and osmotic shock process, was compared with a conventionally commonly used production method. The production method used in the experiments is schematically depicted in Figure 5.

The specific experimental method was as follows:

### Upstream Process

1) A recombinant adeno-associated virus expression plasmid was prepared according to Experimental Example 1.1.
2) Host cells were prepared according to Experimental Example 1.2.
3) The recombinant adeno-associated virus expression plasmid prepared in the step 1) was transformed into the host cells prepared in the step 2) according to Experimental Example 1.3.
4) After performing the process of the step 3), a compound was added 4 hours later.
5) One day after transformation, the pre-culture treatment was performed on the host cells. Depending on the examples, the pre-culture treatment was omitted.
6) Four days after transformation, the cell treatment process was performed for a downstream process. Depending on the examples, the cell treatment process was performed by i) freezing and thawing or performing an osmotic shock (see Experimental Example 1.4 or Experimental Example 1.5).
7) After the treatment in the step 6), the downstream process was performed.

### Downstream Process

1) A resulting product of the upstream process was filtered using a 0.45 µm PES filter followed by precipitation with 40% PEG 8000 under stirring at 4°C for 1 hour and filtration overnight at 4°C.
2) The filtered resulting product was centrifuged at 3000 g at 4°C for 20 minutes.
3) Benzonase treatment was performed on a supernatant obtained as a result of the centrifugation in the presence of 2 mM MgCl₂ at 50 units/mL, 37°C, 45 minutes.
4) The resulting product after the benzonase treatment was centrifuged at 2500g at 4°C for 30 minutes.
5) The ultracentrifugation method according to Experimental Example 1.6 was performed on a centrifugation resulting product.
6) The resulting product obtained from the step 5) was subjected to silver staining according to Experimental Example 1.8, Coomassie brilliant blue staining according to Experimental Example 1.9, and qRT-PCR according to Experimental Example 1.10. The characteristics of the resulting product for each process were analyzed.
7) The resulting product of the step 5) was finally formulated using a 100K PES concentrator, and were finally analyzed according to Experimental Examples 1.7 to 1.12.

The recombinant adeno-associated virus production method is shown in the following table:

**[Table 5]**

| Label | Pre-culture treatment | cell treatment prior to downstream process | downstream process |
|---|---|---|---|
| Process 1 | X | X | X |
| Process 2 | X | freezing and thawing | O |
| Process 3 | X | osmotic shock | O |
| Process 4 | O | X | X |
| Process 5 | O | freezing and thawing | O |
| Process 6 | O | osmotic shock | O |

### Experimental Example 4.2. Comparison of Cell Viability For Each Method

The results of comparing cell viability through TEM analysis immediately after the upstream process of Experimental Example 4.1 for each example are shown in Figures 6 to 8.

As a result of the experiments, Process 2 (15.8%) and Process 5 (18.2%), which included the freezing and thawing process, showed significantly lower cell viability than Process 6 (35.4%), which included the osmotic shock process. This showed that a lot of damage was done to the cells during the freezing and thawing process, and when the osmotic shock process was included, much less cell damage occurred compared to the typical freezing and thawing process.

### Experimental Example 4.3. Yield Comparison for Each Method

The yields of recombinant adeno-associated virus acquired as a result of performing all the processes of Experimental Example 4.1 for each example are shown in Figures 9 and 10 and in the following table.

**[Table 6]**

| Process | Supernatant after filtration and PEG | Cell pellet after filtration and PEG | Supernatant after adding benzonase | Cell pellet after adding benzonase | Final yield after ultracentrifu gation |
|---|---|---|---|---|---|
| | precipitati on (vg) | precipitati on (vg) | (vg) | (vg) | (vg) |
| Process 1 | 1.42E+13 | | | | - |
| Process 2 | 3.48E+13 | 2.86E+13 | 2.80E+13 | 5.75E+12 | 2.70E+13 |
| Process 3 | 3.21E+13 | 1.96E+13 | 3.20E+13 | 1.12E+12 | 2.28E+13 |
| Process 4 | 1.02E+14 | | | | - |
| Process 5 | 1.82E+14 | 1.63E+14 | 1.76E+14 | 9.21E+12 | 1.83E+14 |
| Process 6 | 1.73E+14 | 1.69E+14 | 1.76E+14 | 5.96E+12 | 1.97E+14 |

Experimental results showed that when the pre-culture treatment process was included during cell culture, the host cells produced a large amount of recombinant adeno-associated virus, regardless of the recombinant adeno-associated virus recovery methods.

### Experimental Example 4.4. Purity Comparison of Recombinant Adeno-associated Virus Acquired by Each Method

For each example, the results of Coomassie brilliant blue staining to compare purity are shown in Figures 11 to 13.

Referring to Figures 11 to 13, it was confirmed that purity was significantly lower when acquiring a recombinant adeno-associated virus through the freezing and thawing process (Process 2 and Process 5) than when acquiring the recombinant adeno-associated virus through the osmotic shock process (Process 3 and Process 6). Specifically, when comparing Process 2 and Process 3, it was confirmed that a column appearing in Process 2 showed a significantly higher background band than a column appearing in Process 3. This was believed to be because when the freezing and thawing process was included, the cells were disrupted, and a lot of cell debris was generated.

### Experimental Example 4.5. Genome Integrity Comparison of Recombinant Adeno-associated Virus Acquired by Each Method

For each example, the results of genome integrity performed according to Experimental Example 1.10 are shown in the following table:

**[Table 7]**

| Process | ITR | bGH | Genome integrity |
|---|---|---|---|
| Process 2 | 3.49E+13 | 2.03E+13 | 58% |
| Process 3 | 1.86E+13 | 1.38E+13 | 75% |
| Process 5 | 3.09E+13 | 1.68E+13 | 54% |
| Process 6 | 2.74E+13 | 1.75E+13 | 64% |

The experimental results showed that when recombinant adeno-associated virus was prepared according to the examples in which the osmotic shock was applied, the genome integrity was higher than when recombinant adeno-associated virus was prepared by disrupting cells using the freeze and thaw method. The same trend was shown even when the pre-culture treatment process was not included. This suggested that the osmotic shock process was an important process for producing the intended recombinant-adeno-associated virus in high quality.

### Experimental Example 4.6. Full Capsid-empty Capsid Ratio Comparison of Recombinant Adeno-associated Virus Acquired by Each Method

For each example, the results of TEM analysis performed according to Experimental Example 1.11 are shown in Figures 14 to 17.

As a result of the analysis, the Full/Empty ratio was only 40% and 67% in Process 2 and Process 5, respectively, in which cells were disrupted through freezing and thawing. In comparison, in Process 3 and Process 6, including the osmotic shock process, high Full/Empty ratios of 82% and 87%, were shown, respectively. This showed that the osmotic shock process had a very large effect on the increase in the Full/Empty ratio (compare Process 2 vs. Process 3, and Process 5 vs. Process 6). This showed that the pre-culture treatment process also contributed to some extent to an increase in the Full/Empty ratio (compare Process 2 vs. Process 5 and Process 3 vs. Process 6).

### Experimental Example 4.7. Yield and Purity Comparison of Recombinant Adeno-associated Virus Acquired by Each Method

The yield results of performing the entire process of Experimental Example 4.1 are shown in the following table:

**[Table 8]**

| Process | vg/ml | Total vg | Volume | Percent |
|---|---|---|---|---|
| Process 2 | 7.82E+13 | 2.35E+13 | 0.3ml | 100% |
| Process 3 | 5.15E+13 | 1.54E+13 | 0.3ml | 66% |
| Process 5 | 8.02E+13 | 8.02E+13 | 1.0ml | 342% |
| Process 6 | 8.58E+13 | 8.58E+13 | 1.0ml | 366% |

Additionally, to compare the purity of the acquired recombinant adeno-associated virus, the results of silver staining performed according to Experimental Example 1.8 are shown in Figure 18.

As a result of the experiment, it was confirmed that 1) a significantly higher yield was confirmed in the case of pre-culture treatment compared to the process without pre-culture treatment, and 2) In the case of a process using the osmotic shock process, it was possible to acquire recombinant adeno-associated virus particles in significantly high purity compared to a process including a conventional freezing and thawing process. In conclusion, the method of producing recombinant adeno-associated virus disclosed herein included a pre-culture treatment process and an osmotic shock process. By including the processes, the method might produce a recombinant adeno-associated virus in high quality and high yield compared to conventional production methods.

### Industrial Applicability

The method for producing a recombinant adeno-associated virus disclosed herein can be used to produce a recombinant adeno-associated virus in high quality and high yield. The method is a more excellent and economical method of producing the recombinant adeno-associated virus than conventional production methods.

## Claims

1. A method for producing a recombinant Adeno Associated Virus(rAAV), comprising:
(a) preparing host cells,
wherein the host cell comprises a vector encoding components of the rAAV, such that the host cells are capable of expressing the rAAV;
(b) adding sugar, Nocodazole and M344 to a medium comprising the host cells;
(c) culturing the host cells,
wherein the culturing enables the expression of the rAAV within the host cells;
(d) applying an osmotic shock by adding salt to the media comprising the host cells,
wherein the osmotic shock facilitates the release of the rAAV from the host cells into the medium; and
(e) obtaining the rAAV from the medium without lysing the host cells.

2. The method of claim 1,
wherein a serotype of the rAAV is selected from one or more of the following:
wild-type serotypes including AAV1, AAV2, AAV3, AAV4, AAV5, AAV6, AAV7, AAV8, AAV9, and AAV10;
synthetic (variant) serotypes including AAV-DJ, AAV-DJ8, AAV-DJ9, and AAV6.2; and
other AAV variant serotypes.

3. The method of claim 1 or 2,
wherein the step (e) comprises:
(e-1) separating the medium from the host cells in the product of step (d);
(e-2) filtering the medium;
(e-3) performing a PET precipitation on the product of step (e-2);
(e-4) adding benzonase to the product of step (e-3) in the presence of a salt;
(e-5) performing a 1st iodixanol gradient ultracentrifugation on the product of step (e-4); and
(e-6) performing a 2nd iodixanol gradient ultracentrifugation on the product of step (e-5).
